(19) Europäisches Patentamt

European Patent Office

Office européen des brevets

(11) **EP 3 010 400 B1**

(12) **EUROPEAN PATENT SPECIFICATION**

(45) Date of publication and mention
of the grant of the patent:
**31.07.2019 Bulletin 2019/31**

(21) Application number: **14744429.3**

(22) Date of filing: **18.06.2014**

(51) Int Cl.:
***A61B 5/00*** *(2006.01)*

(86) International application number:
**PCT/CZ2014/000068**

(87) International publication number:
**WO 2014/202033 (24.12.2014 Gazette 2014/52)**

(54) **A METHOD OF DETERMINING SYSTOLIC AND DIASTOLIC BLOOD PRESSURE AND THE UNIT FOR THIS METHOD**

VERFAHREN ZUR BESTIMMUNG DES SYSTOLISCHEN UND DIASTOLISCHEN BLUTDRUCKS UND EINHEIT FÜR DIESES VERFAHREN

PROCÉDÉ POUR DÉTERMINER UNE PRESSION SANGUINE SYSTOLIQUE ET DIASTOLIQUE, ET UNITÉ POUR CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2013 CZ 20130478**
**24.06.2013 CZ 20130488**
**23.05.2014 CZ 20140356**
**02.06.2014 CZ 20140375**

(43) Date of publication of application:
**27.04.2016 Bulletin 2016/17**

(73) Proprietor: **Czech Technical Univerzity in Prague, Faculty of**
**Electrical Engineering, Department of Telecommunication Engineering**
**16627 Praha 6 (CZ)**

(72) Inventors:
• **CHOD, Jiri**
**15800 Praha 5 (CZ)**
• **ZAHRADNIK, Pavel**
**1300 Praha 3 (CZ)**

(74) Representative: **Kratochvil, Vaclav**
**Patent and Trademark Office**
**P.O. Box 26**
**295 01 Mnichovo Hradiste (CZ)**

(56) References cited:
**US-A1- 2006 122 517      US-A1- 2009 326 393**
**US-A1- 2010 168 531      US-A1- 2012 022 382**

**Description**

Technical Field

The invention concerns the method of determining systolic and diastolic blood pressure and the unit enabling to carry out this method. It is the technological solution for determining the systolic and diastolic blood pressure that enables monitoring and measuring humane life functions and utilizes the telecommunication technology with electronics and sensors, computer technology and the wireless communication, factually the solution concerns the unit for monitoring and measuring human life functions by a specifically adapted electronic module and its communication with other devices.

Background Art

Blood pressure is one of the most important physiologic parameters of a human organism. Its measuring belongs among routine procedures in medicine and is a part of most of the medical check-ups.

Considering the fact that around 50 % adults out of adult population suffers from the high blood pressure (hypertension), or they are at the stage of prehypertension, there arises a need of frequent control of real values of the blood pressure, because the deviation from the optimal values can usually bring serious health problems, oftentimes fatal. According to WHO (World Health Organisation) charts and ESH (European Society of Hypertension) charts, the optimal blood pressure is lower - in both values, than 120/80 mmHg. The values between this border and the blood pressure of 139/89 mmHg are considered as prehypertension. The higher values on the other hand are considered as different stages of hypertensions.

**Tab 1: Categories of blood pressure (source WHO/ESH)**

| Categories | systolic BP [mmHg] | diastolic BP [mmHg] |
|---|---|---|
| Optimal BP | < 120 | < 80 |
| Normal BP | < 130 | < 85 |
| Prehypertension | 130 - 139 | 85 - 89 |
| Hypertension | | |
| Modest | 140 - 159 | 90 - 99 |
| Border | 140 - 149 | 90 - 94 |
| Medium serious | 160 - 179 | 100 - 109 |
| Serious | $\geq$ 180 | $\geq$ 110 |
| Isolated systolic | $\geq$ 140 | < 90 |
| Border systolic | 140 - 149 | < 90 |

It is obvious that measuring the blood pressure is more than needful. With this is connected a new approach to control and monitoring of human health E-Health, whose aim is not only to monitor for example post-operation condition, individuals with health risks, but at the end it would be appropriate to monitor the whole population to prevent critical situations. This monitoring, methods usually connected with the complex of other measurements - temperature, heartbeat, breathing, oxygen content in blood etc., require finding such a method that burdens and obtrudes the subject as little as possible. These units for reading quantities can by either autonomous or connected with the assessment center and its assessment. Reading of quantities itself can be either continual or one-time, or both, usually is motivated from the measured subject. It is thus optimal if the device is a part and/or is connected to a modern mobile terminal such as SmartPhone.

Up to date solutions for determining the values of systolic and diastolic blood pressure are based on principles that can be divided into two categories, these are invasive and non-invasive methods.

The invasive method means inserting the reading component directly into the measured bloodstream and it is not relevant for the aim of this solution.

The non-invasive method can be divided to discrete methods - auscultation method, oscillometric method, palpation method, infrasound method, ultrasound method etc. and continuous methods - the vascular unloading method, the method of arterial stiffness, method of sensing of the speed of the pulse wave, etc.

All the above mentioned methods use the cuff either for a wrist or for an arm and afterwards they assess the values depending on the status of inflating this cuff.

An important fact is that with an exception of invasive methods, which obtrude the subject though, the majority of measurements evince a relatively considerable mistake, especially as far as the relation to health condition and the age of a subject is concerned. Considering this it is therefore optimal to do the measurements frequently and assess the changes, or eventually undertake a calibration. As a matter of fact it requires such a principal of measuring systolic and diastolic blood pressure that obtrudes the subject as less as possible or not at all, and all the procedures with the inflating cuff are therefore inconvenient.

The most used method out of the separate methods is the oscillometric method which is based on an assessment of oscillometric pulsations - pressure pulsations that are generated in the inflating cuff during its inflation and deflation. The crucial problem of this method is the still unclear criteria for assessing systolic and diastolic pressure. These values are usually determined by an application of the mathematic criteria on the envelope of oscillometric pulsations. Each manufacturer uses his own secret algorithms and therefore it is impossible to objectively find out sufficient accuracy and repeatability of measurements. Considering the energetic requirements and especially practical application, it is logical, that the measuring by means of inflatable cuff is not possible for continual monitoring of the subject. The same is true about other cited methods.

Another crucial problem of the currently used methods applying inflating cuffs is that older individuals and individuals with damaged blood stream, e.g. diabetes, where the bloodstream tends to return slowly to rest regime and the measurement is therefore not repeatable.

Usually it is not possible to inflate the cuff during the usual activity - it is not possible because of the technological, psychological and social point of view. The solution thus has to be non-invasive und unobtrusive.

To control the continuous but also one-time life functions - e.g. the heartbeat, temperature, breathing, the amount of oxygen in blood, systolic and diastolic pressure, if needed other quantities like ECG, EEG etc., the sets of specialized apparatus are used that only lately began to feature wireless interfaces enabling a connection to other devices such as personal computers, tablet and/or the terminal such as SmartPhone and/or databases and analytical programs.

There are many defined and standardized protocols for communication, that are possible to use for communication and transfer of measured data from the sensor network to the internal computer unit or for the transfer of the data to a remote network. It is namely Bluetooth (IEEE 802.15.1), for the communication at close distance, further GSM/HSCSD/GPRS, UMTS, LTE, WiFi (IEEE 802.11). At the same time there are in common usage systems GNSS - GPS, GLONASS, GALILEO, to determine location and time. There is a preference for the communication module of the type of Bluetooth (IEE 802.15.1) which is characterized by an autonomous control of its surrounding with a corresponding reaction to a call for communication when it switches from a standby mode with a very low consumption to a mode of communication and after the exchange of the data it returns back to a standby mode.

The sensor units are crucial for monitoring which enable contactless collection of data and their transfer to superordinate facilities.

However, the current apparatus for monitoring and measuring the human life functions use only some of the above mentioned standards and that only separately. They represent therefore separate, relatively large-sized units utilized mostly to a measurement of one quantity. The first apparatus that combine more functions appear either in the field of satellite navigation, where they together with another measured quantity, usually heartbeat, serve as a tool for sport activity and/or are implemented as single-purpose ones to monitor one of the life functions' parameters. Typical examples are units for measuring the content of oxygen in blood. In these cases they are usually solved as a cuff and/or finger-mounted module or earlobe-mounted module and the collected data are subsequently transported either by overcrossing wiring and/or wirelessly to the next unit. These units usually feature their own display and operating components. Their main disadvantage is that not only they are not utilized to long-term measurement, but also their high energy consumption demands large-sized power supplies and doesn't allow long-term measurements - in this case it is basically a question of the future technology though. The second main disadvantage is that units are not adjusted for long-term wearing and therefore are not practical as far as any activity other than measuring is concerned.

US2009326393 discloses the determination of blood pressure of a patient based on pulses in a PPG signal. The blood pressure is determined by measuring the area under a pulse or a portion of the pulse in the PPG signal. These measurements are correlated with empirical blood pressure data (corresponding to previous blood pressure measurements of the patient or one or more other patients) to determine the systolic, diastolic and mean arterial pressure.

Disclosure of Invention

The above mentioned drawbacks are eliminated by the method of determining systolic and diastolic blood pressure from parameters of plethysmographic curve according to claim 1 without the necessity of applying the inflating/deflating cuff supplemented with the technical device for monitoring and measuring human life functions that utilizes the telecommunication technology with electronics and sensors, computer technology and the wireless communication, factually the solution concerns the unit for monitoring and measuring human life functions by a specifically adapted electronic module and its communication with other devices.

The default point is the plethysmographic curve. The plethysmographic curve is obtained on the basis of passing through a tissue, so called transmission way of rays of light, while using the reflective method it is possible to work with the reflected light energy. Usually the radiance in the region of the infrared light is used. The device usually houses the light source, scanning element, for example photodiode, phototransistor, camera, etc. and an assessment that can display the transmitted information and/or transfer it for further processing. The plethysmographic curve can be obtained also as another product while measuring the oxygen content in blood. Commonly radiation through a tissue by two different rays of light, usually in the red and infrared regions of spectrum is used.

The plethysmographic curve is digitized and undergoes the filtering and segmentation with the consequent choice of valid segments, and the values of systolic and diastolic blood pressure of the measured subject are determined from the valid segments.

The principle of the invention is that continuously or one-time obtained plethysmographic curve is processed by the procedure according to the invention and resulting values are proclaimed as systolic and diastolic blood pressure of the measured subject - a human.

If the systolic and diastolic blood pressure is determined in a way described according to this invention, the set of obtained data of the plethysmographic curve is divided into separate segments that respond to one heartbeat of the measured subject and they represent one cycle of the heart activity where each segment is defined as two local minimums determined by the beginning and the end of one global maximum between them, with the advantage of 20 segments. In order to eliminate the saturated signal from further evaluation, the minimum and maximum values of the main maximum in relation to saturation of the signal determining the constant $K_a$ and its minimal value $K_{aMIN}$ and maximal value $K_{aMAX}$ and the saturated parts of the plethysmographic curve are excluded from further assessment. The constant $K_a$ is with the advantage 10 for minimal value $K_{aMIN}$ and with the value 95 % of the total maximum in values according to the type A/D converter of the transfer for maximal value $K_{aMAX}$. The course of the left segments of each section of the plethysmographic curve is filtered by calculating means of subsequent values of the plethysmographic curve. The number of segments used for calculation of the mean is related to the used monitoring element in such a way that no significant distortion of the final shape arises and it defines the constant $K_{prum}$, and in each section the minimal and maximal value is determined. The maximal value in each section is matched with the value 1, whereas the minimal value is matched with the value 0. All other values of the pletysmographic curve, with respect to each section, are linearly transformed into a dynamic range 0 to 1. In each section the set of the plethysmographic curve data is divided into individual segments which correspond to one heartbeat of the measured subject and represent one cycle of the heart activity where each segment is defined as two local minimums set by the beginning and the end and one global maximum between them, with the advantage up to 20 segments.

Segments, one independently on the other, are normed as follows: the value Y of the global maximum is matched with the value 1 and the value 0 is determined as a result of the arithmetic mean of the beginning and the end of the segment for the segments with the difference lower than 5 % and the samples are linearly transformed according to those limits and/or the values of the plethysmografic curve for segments with the difference lower than 25 % are recalculated so that the beginning and the end of each segment has the same value 0 and this defines the shift in the offset value and the multiplicative constant gain and chosen segments are again linearly transformed using values offset and gain and the area of each chosen segment is divided into 6 parts defined as dividing vertical from the maximum and divided in the axis Y for values between 20 to 60 % and 40 to 90 % out of maximum, where the second dividing must be percentage larger than the first one. In chosen segments the steepness of systolic run-up is determined as the first derivation of an edge of each chosen segment. Then areas and centers of gravity of all 6 parts are calculated and further closed areas and/or a line are created from the points of centers of gravity. Further total areas and centers of gravity for the selected segment are determined as well as the area and the center of gravity of the new area created from centers of gravity of segment partial areas and/or the size of the line determined by partial points and/or the same in combination with the center of gravity of the whole plethysmographic curve. At the same time the constant $K_{ind}$ is determined as the value of the statistic mean of samples of checking measurements of subjects for systolic and diastolic blood pressure and the constant $K_n$ as the value of the statistic mean of samples of checking measurements for systolic and diastolic blood pressure of the subjects, divided according to age and gender categories, and identically the constant $K_R$ is determined in the same manner as the constant of the basic range segmented for systolic and diastolic pressure, and the systolic pressure is determined as a product of

$$K_{RS} * K_{nS} * K_{indS} * D * S8$$

or

$$K_{RS} * K_{nS} * K_{indS} *D*ST67810$$

or

$$K_{RS} * K_{nS} * K_{indS} *D*(TyST67810* TxST67810)$$

furthermore diastolic pressure as the product of

$$K_{RD} * K_{nD} * K_{indD} *((U911)*S)$$

or

$$K_{RD} * K_{nD} * K_{indD} *(\sqrt{((Tx9-Tx11)^2 + (Ty9-Ty11)^2)} * S)$$

together with determining the diastolic pressure as the product of

$$K_{RD} * K_{nD} * K_{indD} *((TyST*TxST)*S)$$

or

$$K_{RD} * K_{nD} * K_{indD} *STT$$

or

$$K_{RD} * K_{nD} * K_{indD} *(TySTT*TxSTT)$$

The segment must be longer than minimal and shorter than maximal allowed limit, while limits are defined in time constants equivalent to number of assessed pulses of heartbeat starting with 5 pulses as the minimum up to 250 pulses as the maximum.

Determining the number of samples used for calculating of the mean is defined advantageously as the constant $K_{prum}$, this constant is determined according to the type of scanning unit in relation to actual disturbance of its amplifier and it represents a compromise in a sense that it smoothes desirably a signal and it still does not undesirably and considerably distort the signal, especially its rapid changes in the vicinity of local extremes. The value of the constant $K_{prum}$ is with the advantage in the range 2 to 30 in terms of consecutive samples.

The constant $K_R$ is the constant of the basic range of systolic pressure and is with an advantage determined as $K_{RS}$ for systolic pressure with the basic value $K_{aMIN}/10$ and it is with an advantage determined as $K_{RD}$ for diastolic blood pressure with the basic value $K_{aMIN}/100$.

The division into partial areas 6, 7, 8, 9, 10, 11 is with an advantage determined as 35 % and 55 % out of maximum.

The constant $K_{ind}$ equals implicitly 1 for systolic and diastolic blood pressure.

The significant advantage according to the invention is that scanning the plethysmographic curve is entirely non-invasive, without any sound effects, energetic requirements are lower than typical compressor and its electromotor. The significant advantage also is that the device does not include any movable parts. Its usage is in terms of health harmless and can be applied for arbitrarily long period. There is a reasonable assumption that especially with the older subjects and subjects with damaged blood stream, the final determining of systolic and diastolic blood pressure is more accurate and the resulting measurements are repeatable as they don't influence the status of the blood stream in any way.

The above mentioned drawbacks are considerably eliminated by the unit for monitoring and measuring human life functions, according to claim 12. It is based on a carrier construction in a shape of a ring and/or an earring equipped with a transmitting sensor, another transmitting sensor, scanning sensor for transmitting sensor, other transmitting sensor and scanning sensor for measuring temperature of surrounding, that are connected via interface to a communication module unit wirelessly connected to another communication module placed out of the ring and/or the earing and interconnected to a supervising controlling unit, at the same time the transmitting sensor, another transmitting sensor and other transmitting sensor, scanning sensor for measuring temperature of surrounding and the communication module unit are powered from battery implemented in the ring which is placed on the fingertip and/or the earlobe of the monitored and measured subject.

The transmitting sensor can be furnished with an infrared radiation supply for the plethysmographic curve data supply of the measured subject scanned by the scanning sensor and/or infrared radiation supply for the plethysmographic curve supply scanned by the scanning sensor.

The networks made of sensors are connected to each other directly to the communication module via its interface and then wirelessly, using the network of short coverage, the data are transferred to another device, all the electronics is integrated in a shape of the ring and/or the earring and it is adapted for long-term measurements and wearing, at the same time, the data assessments as well as the other calculations are centralized into a superordinate unit, which is of any type, but has its communication unit.

The invention is based on a communication module unit connected via interface for communication module connection to both, the transmitting sensor and to another transmitting sensor at the same time and further it is attached to the scanning sensor and another scanning sensor for measuring temperature of the ring surrounding and/or the surrounding of the earlobe inside the earring, all the modules are supplied from the inbuilt battery and they are places inside the mechanical construction that forms the ring and/or the earing put on a finger and/or an earlobe of the monitored and measured subject - a human, the modules wirelessly transmit and receive data by means of the communication module to the superior controlling, assessing and computing unit.

The main construction advantage is the simplicity of electronic blocks that at the end enables a simple processing of the construction of the ring and/or the earring itself and its design, the design can even follow the fashion jewellery. The construction of installation of the scanners in a shape of the ring and/or the earring ensures reliable transit transition of measured signals and their assessment from the point of view of measured human tissue. As a consequence these are not that critical movements of measured subject in the course of the measurement and the measurement itself is more stable and accurate.

Explanation of Figures in the Drawings

The invention will be described in more detail in particular examples and described in enclosed drawings, where the Fig. 1 shows the plethysmographic curve - the example of the data set. The Fig. 2 shows the segments in the course of the plethysmographic curve. The Fig. 3 is an example of two valid normalized selected segments of the plethysmographic curve. The Fig. 4 is an example of two valid normalized and selected segments of plethysmographic curve and its division into partial segments. The Fig. 5 is an example of two valid normalized segments of the plethysmographic curve in the layout of connected lines M1, M2, M3 and its division of the partial segments. The Fig. 6 states the features evaluated from the plethysmographic curve and the Fig. 7 shows the features evaluated from the plethysmographic using new areas determined from centers of individual segments and the total center. The technical solution is explained in more detail in the enclosed drawing, where in the Fig. 8 for the ring construction and in the Fig. 9 for the earring construction, there is an overview scheme of solution and communication and it also shows the block scheme of a sensor network and communication modules. The Fig. 10 shows an example of communication of the unit that provides the systolic and diastolic blood pressure determination in cooperation with the surrounding environment.

Examples of Invention Implementation

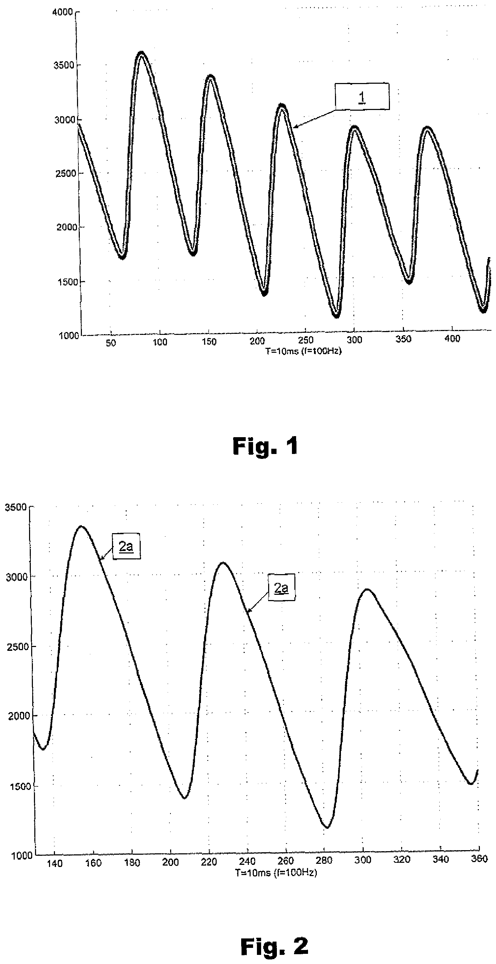

An example of the way of determining systolic and diastolic blood pressure according to the invention is described in a particular example of processing - Fig. 1 to 7.

The set of data obtained from separate curves of plethysmographic curves **1** are split into segments **2a** where one segment **2a** corresponds to one heartbeat of the measured subject and it represents one cycle of the heart activity, it is therefore a section of examples of the plethysmographic curve **1** between two local minimums **M1** and **M2** while segment **2a** includes one global maximum **M3** between the beginning and the end of the segment **2a,** the segment **2a** must be longer than the minimal and shorter than the maximal allowed limit, these are defined in constants of time units that are equivalent to a number of evaluated pulses of the heart beat and this course of segments **2a** of the plethysmographic curve **1** is divided into individual sections containing one or more segments **2a** with the advantage of division to sections up to 20 segments **2a.** To exclude the saturated signal from further assessment, the saturated parts of segments **2a** of the plethysmographic curve **1** are excluded from further assessment according to the constant $K_a$ and the left segments **2a** are filtered by calculating the mean of subsequent curve sample values according the constant $K_{prum}$. Each segment **2a**, one each independent on the other, is checked from the point of view of the pertinence of measured data in the manner that the differences of values of the Y coordinate of the beginning **M1** and the end **M2** of the curve are checked and only left selected segments **2b** are standardized, one each independent on the other in a way, that Y value of the global maximum **M3** is assigned with the value 1 and after the 0 value is determined as a result of the arithmetic mean of the beginning **M1** and the end **M2** of the selected segment **2b** afterward the samples are linearly transformed considering these values obtained this way and/or the values for the pletysmographic curve **1** for segments **2a** with the difference between the beginning **M1** and the end **M2** lower than 25 % recalculate in a manner, that the beginning and the end of each segment has the same value and selected segments **2b** are again linearly transformed, when the features for parameters of the curve such as the steepness of **D** of the systolic run-up as the first derivation of the leading edge of each selected segment **2b** and the partial areas of each selected segment **2b** are determined, the area of each selected segment **2b** is divided into 6 parts marked **6, 7, 8, 9, 10, 11** and obtained as the dividing vertical from the maximum **M3** of the selected segment **2b** divided in the axis Y for the values 35 % and 55 % out of the maximum **M3** and/or the area of each selected segment **2b** is divided into 6 parts defined as the dividing vertical from the maximum **M3** divided in the axis Y for the values between 20 to 60 % and 40 to 90 % out of the maximal value, where the second dividing line must be by percentage bigger than the first one. Now the total area **S** is determined for each selected segment **2b** and identically the area of each part is determined, which is **S6, S7, S8, S9, S10, S11** together with the center of gravity of each part as well as the whole segment, which means **Tx, Ty, Tx6, Ty6, Tx7, Ty7, Tx8, Ty8, Tx9, Ty9, Tx10, Ty10, Tx11, Ty11** and then for each selected segment **2b** the area **ST67810** is determined using the centers of gravity of parts **6, 7, 8, 10** and/or the centers of gravity of parts **9, 11** that help to create the line **U911 (Ux911, Uy911)**, and by means of the total center of gravity **Tx,y** of the plethysmographic curve **1** the other shapes with the area **STT67810** and its centers of gravity **TxSTT67810, TySTT67810** and the area **STT** and its centers of gravity **TxSTT, TySTT** are created. The constant $K_{ind}$ is determined as the value of statistic mean calculated from a large number of subjects' checking measurements' samples for systolic and diastolic pressure, implicitly it equals 1 and then the constant $\mathbf{K_{ind}}$ is determined from the set of samples of checking statistic measurements, the constant $\mathbf{K_{ind}}$ is divided into a part of the systolic pressure $\mathbf{K_{indS}}$ and the part of the diastolic pressure $\mathbf{K_{indD}}$, together with the constant $\mathbf{K_n}$ determined as the value of statistic mean of the large number of checking measurement's samples divided according to the age and gender category and the condition of the subject's blood stream divided in the part of the systolic blood pressure $\mathbf{K_{indS}}$ and diastolic blood pressure $\mathbf{K_{indD}}$ and the constant $\mathbf{K_{RS}}$ is determined form the basic range of the systolic pressure and the constant $\mathbf{K_{RD}}$ of the basic range of the diastolic range, afterwards the systolic blood pressure is determined as a result of

$$K_{RS} * K_{nS} * K_{indS} *D*S8$$

and at the same time the diastolic blood pressure is determined as a result of

$$K_{RD} * K_{nD} * K_{indD} *((Ty*Tx)*S)$$

where the systolic blood pressure is determined as a result of

$$K_{RS} * K_{nS} * K_{indS} *D*ST67810$$

or

$$K_{RS} * K_{nS} * K_{indS} *D*(TyST67810* TxST67810)$$

and at the same time the diastolic blood pressure is determined as a result of

$$K_{RD} * K_{nD} * K_{indD} *((U911)*S)$$

or

$$K_{RD} * K_{nD} * K_{indD} *(\sqrt{((Tx9-Tx11)^2 + (Ty9-Ty11)^2)} * S)$$

together with determining the diastolic blood pressure as a result of

$$K_{RD} * K_{nD} * K_{indD} *((TyST*TxST)*S)$$

or

$$K_{RD} * K_{nD} * K_{indD} *STT$$

or

$$K_{RD} * K_{nD} * K_{indD} *(TySTT*TxSTT)$$

where scanning of the plethysmographic curve itself is recommended to be done while resting, without any major movement of the measured subject and then according to a need, at the same time other determined parameters such as the total area **S** or the area of each part **6** to **11** marked **S6, S7, S8, S9, S10, S11** and the center of gravity of each part as well as of the total segment **Tx, Ty, Tx6, Ty6, Tx7, Ty7, Tx8, Ty8, Tx9, Ty9, Tx10, Ty10, Tx11, Ty11** are applied, as well as the area **ST67810** and the centers of gravity **TxST67810, TySTT6810** of the new area created from the centers of gravity of the segments' partial areas, respectively the size of the line determined by centers of gravity of partial points marked **Ux911, Uy911,** and /or the same in combination with the total center of gravity, labelled as the area **STT67810** and centers of gravity **TxSTT67810, TySTT67810** and the area **STT** and its centers of gravity **TxSTT, TySTT** in an application for correction of the method of determining the systolic and diastolic blood pressure.

Determining of systolic blood pressure and diastolic blood pressure themselves is taken over from the source - either a separate device comprising the light source irradiating a tissue of the subject and the scanner scanning the final plethysmographic curve, or as the case might be from one channel forming the unit for oxygen content measuring and/or other similar device, the data corresponding to the tissue condition as described in the Fig. 1 and we proceed as follows:

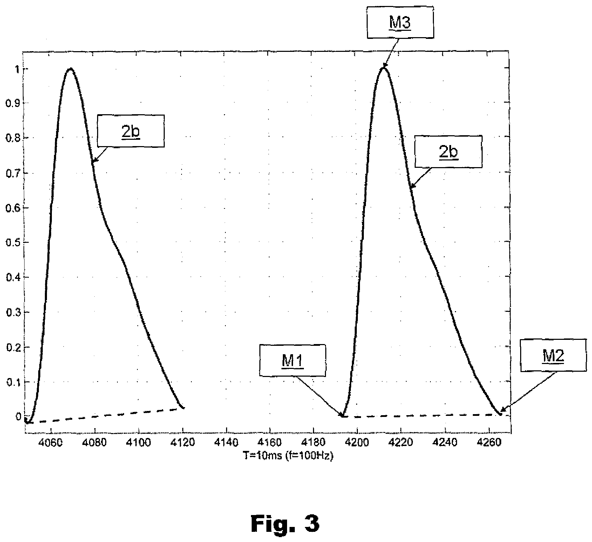

1. The set of received data of each curve - Fig. 1 plethysmographic curves **1** consists of - Fig. 2 segments **2a,** one segment **2a** corresponds to one heartbeat of the measured subject - a human and it represents one cycle of the heart activity. Segment **2a** is therefore a section of plethysmographic curve examples between two local minimums **M1** and **M2** and segment **2a** includes one global maximum **M3** between the beginning and the end of the segment **2a** - Fig. 3. Segment **2a** must be longer than the minimal and longer than the maximal allowed limit. These limits are defined in constants of time units that are equal to number of evaluated pulses of heartbeat, e.g. 5 pulses as the minimum and 250 pulses as the maximum.

2. The course of segments **2a** of the plethysmographic curve **1** is divided into individual sections containing one or more segments **2a** with the advantage of dividing into section up to 20 segments **2a** and to exclude the saturated signal from further assessment the other saturated parts of segments **2a** of the plethysmographic curve **1** are then in accordance with the constant $K_a$ excluded from further processing. Recommended values are always determined according to the type of the scanning unit as the constant $K_a$ or its limits, which means $K_{aMIN}$ and $K_{aMAX}$. See an example in Fig. 1 or Fig. 2, where the recommended value will be $K_{aMIN}$ = 1000 for the minimal value and $K_{aMAX}$ = 4995 for the maximal value. Both values enable exclusion of the saturated segments. The constant $K_a$ advantageously equals 10 for the minimal value $K_{aMIN}$ and with the value that equals 95 % out of the total maximum in values according to the type AID of the transfer for the maximal value $K_{aMAX}$.

3. The course of segments **2a** of the plethysmographic curve is filtered by making an average from the subsequent values of the curve examples. The determination of segment number used for averaging is defined as the constant $K_{prum}$ and is defined according to a type of the scanning unit, in connection to its own interference of its amplifier and it is a compromise in a sense that the signal is desirably purified and at the same time it still doesn't affect the signal significantly, so the rapid changes near the local extremes are not distorted - Fig. 1 and Fig. 2, The typical value $K_{prum}$ ranges for contemporary amplifiers from 2 to 30 subsequent samples.

4. In each section the maximal and minimal values are determined. In each section the maximal value is matched with the value 1 and the minimal value is matched with the value 0. All other values of the plethysmographic curve, with respect to each section, are linearly transformed into a dynamic range 0 to 1.

5. Each segment **2a**, one independently on another, is checked from the point of view of its relevance of measured data. Therefore the differences of value Y and coordinates of the beginning **M1** and the end **M2** of the curve are checked. Only the selected segments **2b** that differ by a defined quantity - e.g. 5 % are left and/or the values of the plethysmographic curve (1) are recalculated in a way that the beginning and the end of each segment's minimum have the same value for differences lower than 25 %. This value corresponds to the final accuracy of a measurement of the resulting pressure. See Fig. 2 and Fig. 3.

6. Left selected segments **2b** are normed, one independently on another in that way, that the Y value of the global maximum **M3** matches the value 1. See Fig. 3.

7. The value 0 is determined as a result of the arithmetic average of the beginning **M1** and the **M2** of the segment for segments with the difference lower than 5 %, and/or the values of the plethysmographic curve recalculated in the way, that that the beginning and the end of each segment's minimum have the same value. This defines the shift in value (offset) and the multiplicative constant (gain).

8. Examples are linearly transformed by values offset and gain (Fig. 3).

9. Features for curve parameters are determined in selected segments **2b**, namely

- the steepness of the systolic run-up is determined as the first derivation of the leading edge of each selected segment **2b** - quantity **D**

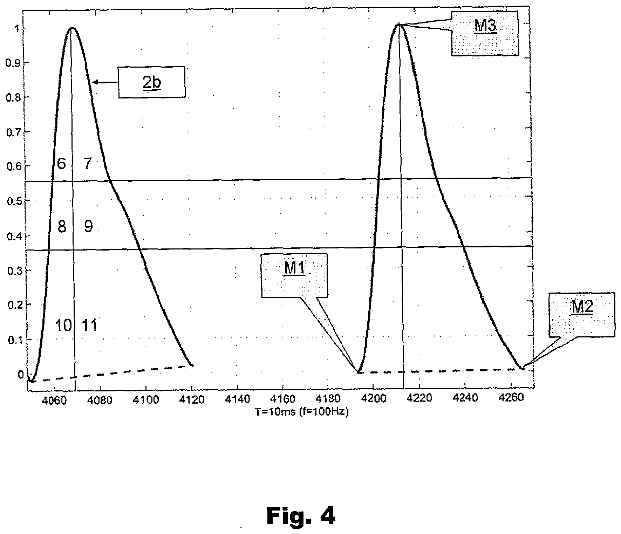

- partial areas of each selected segment **2b** described in Fig. 4, Fig. 5, Fig. 6 and Fig. 7 are determined by dividing the area of each selected segment **2b** into 6 parts defined by a dividing vertical from the maximum **M3** of the segment divided in the axes Y for values 35 % and 55 % out of maximum **M3.**

- partial areas of each segment are numbered according to the Fig. 4, Fig. 5, Fig. 6 and Fig. 7 respectively.

10. For each selected segment **2b** area parameters are determined:

- total area **S**

- area of each part **6** to **11** is numbered **S6, S7, S8, S9, S10, S11**

- the center of gravity of each part and of the whole segment, namely **Tx, Ty, Tx6, Ty6, Tx7, Ty7, Tx8, Ty8, Tx9, Ty9, Tx10, Ty10, Tx11, Ty11**

11. For each selected segment **2b** its parameters of areas and centers of gravity are determined using individual centers of gravity of parts **6, 7, 8, 10:**

- the total area **ST67810** of the inner center of gravity

- the centre of gravity **TxST67810, TyST67810** of this segment.

12. For each selected segment **2b** its area parameters are determined using the individual centers of gravity of parts **9**, **11**:

- line **U911**

13. For each selected segment **2b** its area parameters are determined using individual centers of gravity of parts **6,7,8,10** and the total center of gravity **Tx,y** of the plethysmographic curve:

- total area **STT67810** of the inner center of gravity

- centers of gravity **TxSTT67810, TySTT67810** of this segment.

14. For each selected segment **2b** the area parameters are determined using its individual centers of gravity of parts **9, 11** and the total center of gravity **Tx,y:**

- total area **STT** of the inner center of gravity

- centers of gravity **TxSTT, TySTT** of this segment.

15. The constant $K_{ind}$ is determined as the value of the statistic average of the large number of subject checking measurement examples for systolic and diastolic blood pressure, implicitly it is 1. Its actual value that serves as a correction of value referring to a particular subject can be determined as a result of comparison on checking measurement, laboratory measurement of the subject in comparison with the value stated according to paragraphs 17 to 20. This measurement is carried out only in necessary cases when there is a request for the highest possible accuracy of the resulting determination of systolic and diastolic pressure. The constant $K_{ind}$ is in this case divided into a part of the systolic blood pressure $K_{indS}$ and diastolic blood pressure $K_{indD}$. A standard accuracy of measurement without any individual correction $K_{ind}$ must be better than ± 15 %. It is appropriate to make the measurements at rest, without a move of a subject according to recommended control in accordance with the paragraph 21. The constant $K_{ind}$ is used as a correction for subjects that have vast damages on blood stream that are typical for some diseases such as diabetes etc. It concerns the cases when the standard classification of a subject into one of categories of the constant $K_n$ according to the paragraph 16 is not sufficient.

16. The constant $K_n$ is determined as the value of the statistic average of a large number of subject checking measurement examples divided according to the gender and age category. The constant shows the status of the subject's blood stream in dependence on gender, age, its diseases as the case might be, e.g. diabetes, atherosclerosis etc. The constant $K_n$ is split into a part for the systolic blood pressure $K_{nS}$ and the part for the diastolic blood pressure $K_{nD}$ and that in all following categories of subject classification.
The statistic value is controlled in three basic categories:

- juniors for the age category 10 - 20 years ..... $K_{jun,}$ meaning $K_{junS}$ and $K_{junD}$

- adults for the age category 21- 50 years ..... $K_{st}$ meaning $K_{stS}$ and $K_{stD}$

- seniors for the age category 50 years and over ...$K_{sen}$ meaning $K_{senS}$ and $K_{senD}$

Its value can be affected by the measuring channel that provides the plethysmographic curve, but only as a multiplicative constant.

17. The systolic blood pressure is determined as a result of

$$K_{RS} * K_{nS} * K_{indS} *D*S8$$

where $K_{nS} = K_{junS}$ or $K_{stS}$ or $K_{senS}$ according to a type of the measured subject and his/her age

$K_{ind} = K_{indS}$ in cases specified in the paragraph 15, if the implicit value 1 is not sufficient

D is the steepness of the systolic run-up defined as the first derivation of the run-up of each selected segment **2b** of the plethysmographic curve **1**

$K_{RS}$ is the constant of the basic range of the systolic blood pressure; with advantage it is possible to use the initial value $K_{aMIN}$ /10

18. The diastolic blood pressure is determined as a result of

$$K_{RD} * K_{nD} * K_{indD} *((Ty*Tx)*S)$$

where $K_{nD} = K_{junD}$ or $K_{stD}$ or $K_{senD}$ according to a type of the measured subject and its age
$K_{ind} = K_{indD}$ in case described in the paragraph 15, if the implicit value 1 is not sufficient
$K_{RD}$ is the constant of the basic range, it is possible to use it advantageously as a default value $K_{aMIN}$ /100

19. The systolic blood pressure is determined as a result of

$$K_{RS} * K_{nS} * K_{indS} * D * ST67810$$

and/or

$$K_{RS} * K_{nS} * K_{indS} * D * (TyST67810 * TxST67810)$$

where $K_{nS} = K_{junS}$ or $K_{stS}$ or $K_{senS}$ according to a type of the measured subject and its age

$K_{ind} = K_{indS}$ in case described in the paragraph 15, if the implicit value 1 is not sufficient

$D$ is the steepness of the systolic run-up which is determined as the first derivation of the leading edge of each selected segment 2b of the plethysmographic curve 1

$\mathbf{K_{RS}}$ is the constant of the basic range of the systolic pressure, it is possible to use it advantageously as a default value $\mathbf{K_{aMIN}}$ /10

20. The diastolic blood pressure is determined as a result of

$$K_{RD} * K_{nD} * K_{indD} * ((U911) * S)$$

specifically

$$K_{RD} * K_{nD} * K_{indD} * (\sqrt{((Tx9-Tx11)^2 + (Ty9-Ty11)^2)} * S)$$

and/or

$$K_{RD} * K_{nD} * K_{indD} * STT$$

and/or

$$K_{RD} * K_{nD} * K_{indD} * (TySTT * TxSTT)$$

where $K_{nD} = K_{junD}$ or $K_{stD}$ or $K_{senD}$ according to the type of the measured subject and his/her age

$K_{ind} = K_{indD}$ in cases specified in the paragraph 15, if the implicit value 1 is not sufficient

$K_{RD}$ is the constant of the basic range; beneficially it is possible to use the initial value $K_{aMIN}/100$

21. The acquisition of the plethysmographic curve **1** is recommended to be carried out at rest, without a significant movement of the measured subject. The acquisition is recommended in relation to e.g. a 3D movement sensor with determining its limits

22. Other calculated parameters, specifically:

- total area **S**

- area of each part **6** to **11** numbered **S6, S7, S8, S9, S10, S11**

- center of each part and of the whole segment, namely **Tx, Ty, Tx6, Ty6, Tx7, Ty7, Tx8, Ty8, Tx9, Ty9, Tx10, Ty10, Tx11, Ty11**

- total area **ST67810** created using the centers of gravity of parts **6, 7, 8, 10**

- the centers of gravity **TxST67810, TyST67810** of this area,

- line **U911** created from centers of gravity of parts **9**, **11**

- the area **STT67810** created using the centers of gravity of parts **6, 7, 8, 10,** and the center of gravity **Tx,y,**

- the center of gravity **TxSTT67810, TySTT67810** of this area

- the area **STT67810** created using the centers of parts **9, 11** and the center **Tx,y**, so

- the centers of gravity **TxSTT67810, TySTT67810** of this area

are possible for correction of the manner of calculation according to paragraphs 17 to 20 for specific cases of further more accurate value determination of systolic and diastolic blood pressure on the basis of checking measurement of large number of subjects that have specific features of their blood stream.

Fig. 1 shows an example of the plethysmographic curve **1** taken by the input module and transferred for pressure determination. The curve shows the quality of blood supply in tissues and provides information about the reactivity of the blood circulation. The plethysmography makes it possible to get the record of pulse waves with the help of a sensor and a source of light.

Fig. 2 shows how the plethysmographic curve **1** is divided into segments matching one heartbeat.

Fig. 3 shows how the valid segments of the plethysmographic curve **1** are made depending on value quantity of points **M1** and **M2**.

Fig. 4 shows the split of valid segments into partial segments by determination of the dividing borders in the axis Y.

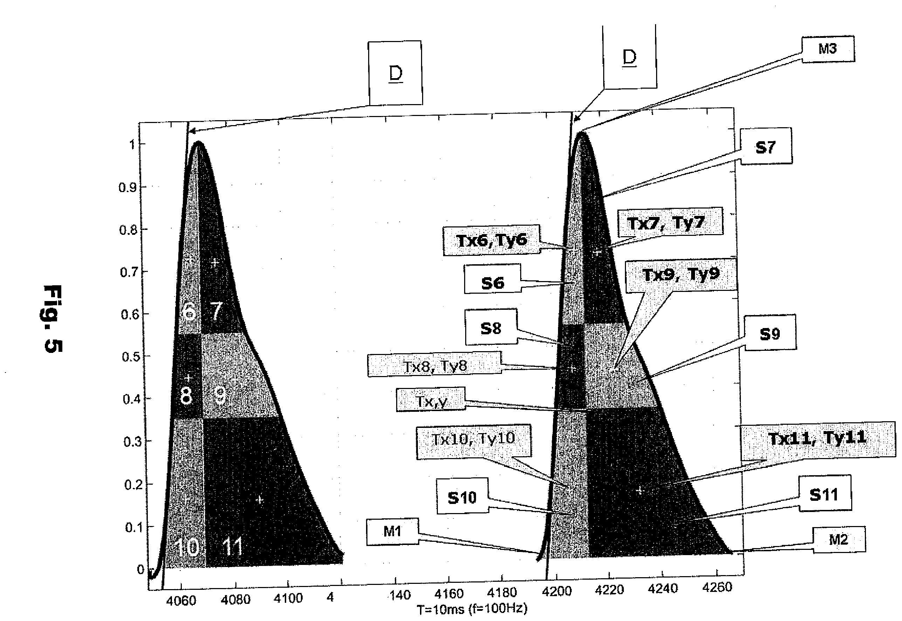

Fig. 5 shows two valid normalized segments of the plethysmographic curve **1,** their labelling of separation into partial segments and determination of areas and centers of gravity.

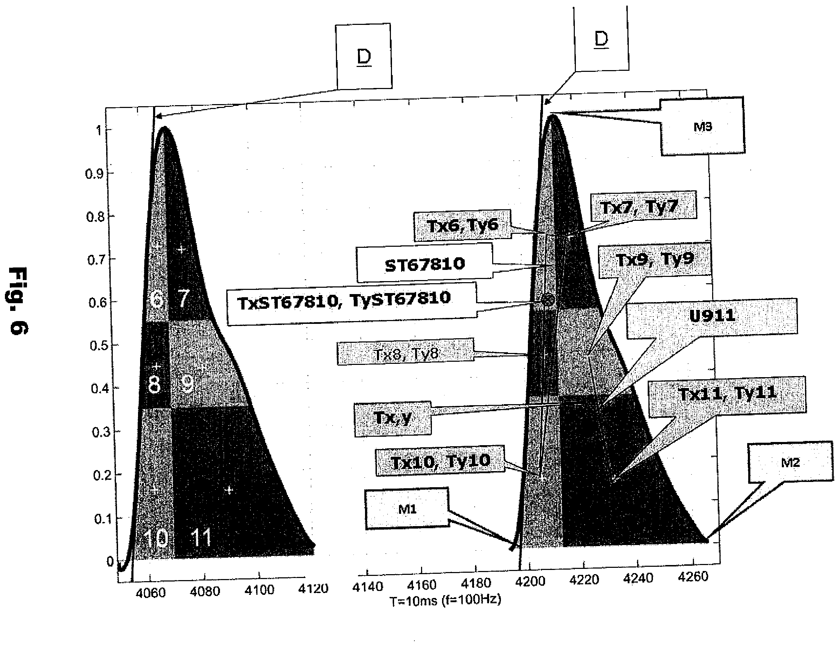

Fig. 6 shows features of the plethysmographic curve **1** where the new areas and a line determined from each segment's centers are used.

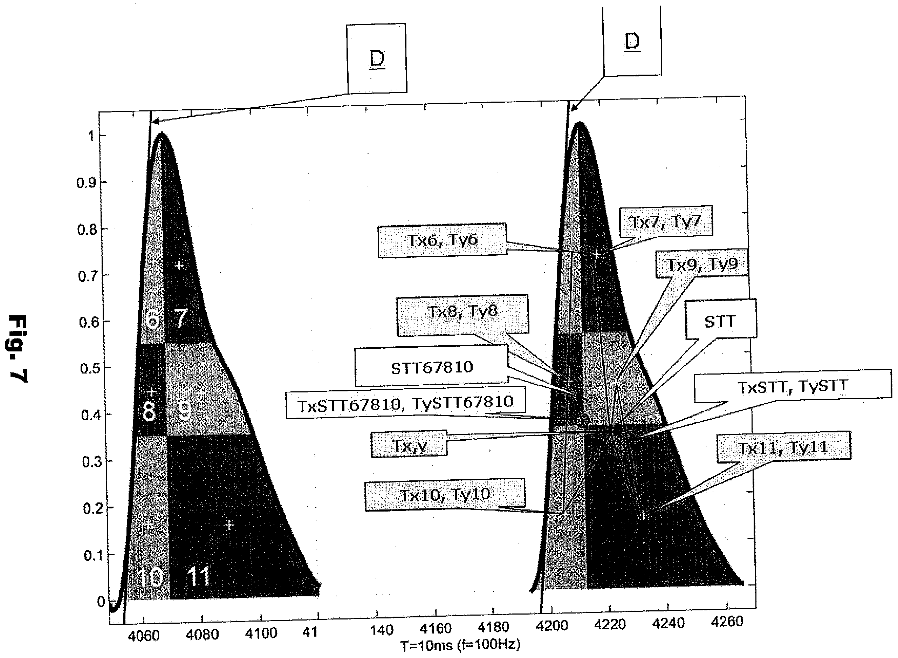

Fig. 7 shows features evaluated from the plethysmographic curve **1,** where the new areas determined from each segment's centers and the total center are used.

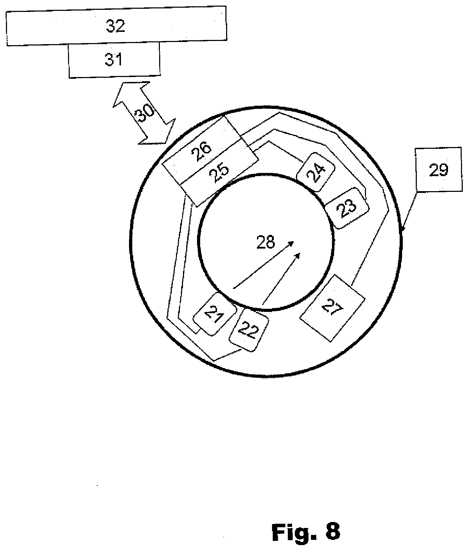

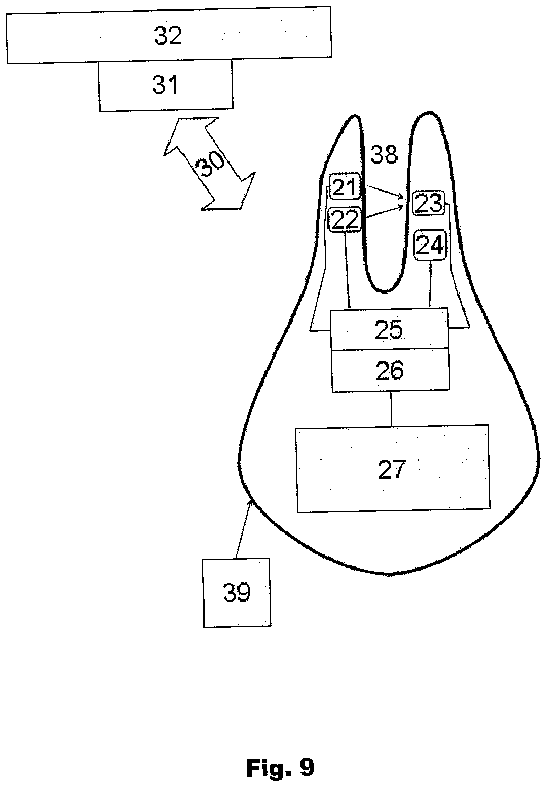

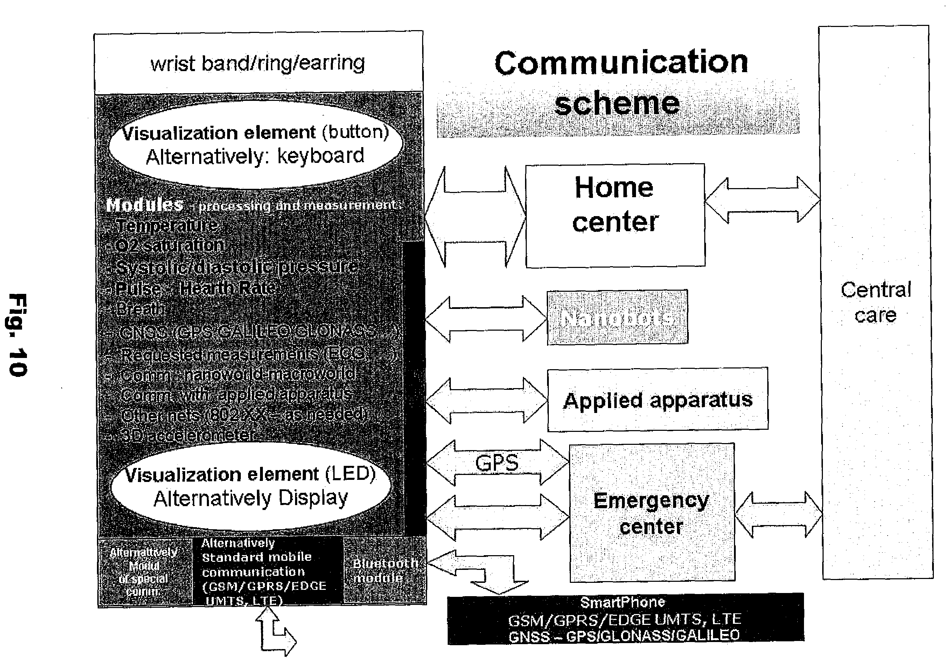

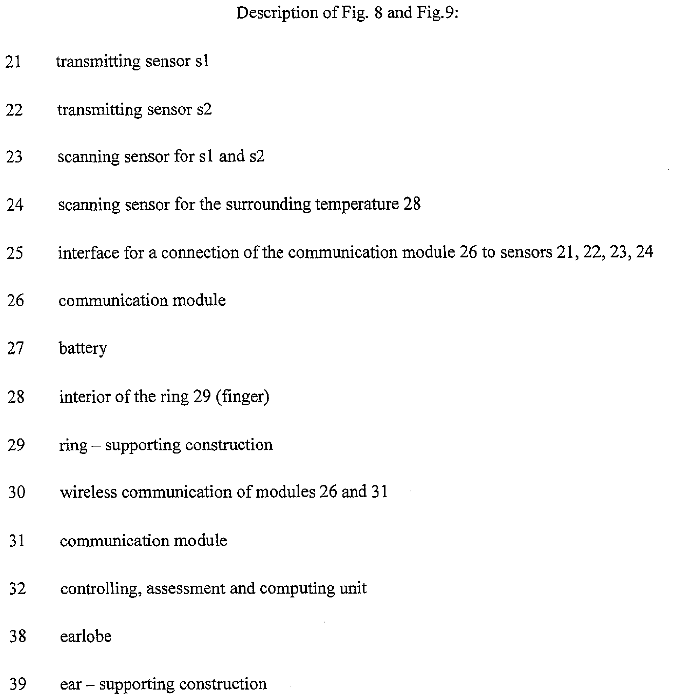

An example of the layout of the unit for monitoring and measuring human life functions according to the invention is introduced as a block in the attached drawings in Fig. 8 and Fig. 9, once for the constructional layout in a shape of the ring - Fig. 8 and once for the constructional layout in a shape of the earring - Fig. 9. Figure 10 is an example of the communication of the unit necessary to determine systolic and diastolic blood pressure in a cooperation with the surrounding environment.
The unit **26** of the communication module is connected over the interface **25** for communication module connection to both, the transmitting sensor **21** and another transmitting sensor **22** and at the same time it is connected to the scanning sensor **23** and also to the scanning sensor **24** for surrounding temperature scanning, while all the modules are supplied from the inbuilt battery **27** and are placed in the space of the mechanical construction that forms a ring **29** and/or an earring **39** put on the finger **28** and/or the earlobe **38** of the monitored and measured subject - a human, and using the wireless communication **30** they transfer and receive data by means of another communication module **31** to the superordinate unit **32**.
During measurement, the assessment and controlling **32** gives an order by which another communication module **31** sets into operation and by the wireless communication **30** the communication module **26** is activated and over the interface **25** for the connection the scanning sensors **23** and **24** are controlled, these modules read the surrounding temperature of the finger **28** or the earlobe **38** as well as the value statuses transmitted by the transmitting sensor **21** and the other transmitting sensor **22** and transfer these data by the interface **25** and communication scanning module **26** and other communication module **31** to the controlling, assessment and computing unit **32.**
By means of this unit **32** it is possible to monitor and measure continuously human life functions such as heartbeat, temperature, breathing, oxygen content in blood, systolic and diastolic blood pressure, or alternatively also other quantities such as ECG, EEG etc., and by wireless interface enables the connection to other devices as personal computer, tablet and/or terminal of the type Smart Phone and the like.
The programming equipment of the assessment and controlling unit **32** processes the data afterwards and displays them in a graphical or numeric shape, alternatively it transfers them to other superordinate units.

The obtained data can be further processed for example to determine oxygen content in blood, number of pulses of the heart activity and/or calculation of systolic/diastolic pressure.

Industrial Applicability

The way of systolic and diastolic blood pressure determination according to this invention will have a wide range of use. It will be employed for usual civil individual care, in the field of the medical care and postoperative monitoring of patient's condition, but especially it will serve as one of the foundations of the E-Health networks for future check-ups of all subjects and that way it will help to prevent critical health conditions.

The integrated system for collecting, processing and distribution of data, especially in the shape of the ring, where the sensor networks are connected by each other directly to the communication module over its interface and further wirelessly, using the short coverage network, are the data transferred to another device, where the set-up is adapted for long-term measuring and wearing, at the same time the complete controlling module and assessment of the data, together with other calculations, are concentrated to a superordinated unit of any type, only equipped with a communication module - controlling unit.

**Claims**

1. A method of determining systolic and diastolic blood pressure, **wherein** the received data set of separate curves of plethysmographic curve (1) is divided into individual segments (2a) with two local minimums given by the beginning (M1) and the end (M2) and one global maximum (M3) that matches one heartbeat of the measured subject and represents one cycle of the heart activity, the course of segments (2a) of the plethysmographic curve (1) is then divided into individual sections containing at least one segment (2a) with the division up to 20 segments (2a) and for the exclusion of the saturated signal from further assessment are according to the constant $K_a$ the saturated parts of segments (2a) excluded from further assessment and the recommended values are always determined according to a type of the scanning unit as the constant $K_a$ respectively its limits, specifically $K_{aMIN}$ a $K_{aMAX}$, and the course of the plethysmographic curve (1) is then filtered by making averages of consecutive values of the plethysmographic curve (1), each segment (2a) is checked for differences between the amplitude of each segment at the beginning (M1) and the end (M2) of the curve and only selected segments (2b) are left, these segments up to 25 %, selected segments (2b) are being normed so that the value (Y) of the global maximum (M3) is matched with the value 1 and the value 0 is determined as a result of the arithmetic average of the beginning (M1) and the end (M2) for selected segments (2b) with the difference lower than 5 % and/or the values of the plethysmographic curve (1) for the selected segments (2b) with the difference lower than 25 % are recalculated in the manner that the beginning and the end of the selected segment (2b) have the same value, and are divided into 6 parts under the curve of a segment defined by dividing the segment in time at its maximum (M3) and dividing the amplitude of the segment by two dividings one between 20 to 60 % and 40 to 90 % out of the maximum (M3), where the second dividing must be by percentage larger than the first one and the areas S6, S7, S8, S9, S10, S11 are determined for each part as well as the centers of gravity Tx6, Ty6, Tx7, Ty7, Tx8, Ty8, Tx9, Ty9, Tx10, Ty10, Tx11, Ty11 of each part and the centers of gravity Tx, Ty and the total area S of the whole selected segment (2b), at the same time the constants $K_{indS}$ and $K_{indD}$ are determined as the values of the statistic average of subject checking measurements example for systolic and diastolic pressure and the constants $K_{nS}$ and $K_{nD}$ as the values of the statistic average of the subject controlling measurements divided according to the age category for systolic and diastolic pressure and that together with a determination of the range constants $K_{RS}$ and $K_{RD}$ of the measurement range for systolic and diastolic pressure, the quantity D is determined as the first derivation of the leading edge of each segment (2a) and now the systolic blood pressure is determined as a result of $K_{RS} * K_{nS} * K_{indS} * D * S8$ and the diastolic blood pressure is determined as a result of $K_{RD} * K_{nD} * K_{indD} * ((Ty*Tx)*S)$.

2. The method according to claim 1, **characterized by** determining the partial areas (S6, S7, S8, S9, S10, S11) and the centers of gravity (Tx6, Ty6, Tx7, Ty7, Tx8, Ty8, Tx9, Ty9, Tx10, Ty10, Tx11, Ty11) of each part and centers of gravity (Tx, Ty) of the whole selected segment (2b) of the plethysmographic curve (1) and using these the new area (ST67810) is determined with its center of gravity (TxST67810, TyST67810) and/or the line (U911) and/or with the whole centers of gravity (Tx,Ty), areas (STT67810) and (STT) with their centers of gravity (TxSTT67810, TySTT67810) and (TxSTT, TySTT) and further the systolic blood pressure is determined as a product of $K_{RS} * K_{nS} * K_{indS} * D * ST67810$ respectively $K_{RS} * K_{nS} * K_{indS} * D * (TyST67810 * TxST67810)$ and at the same time the diastolic blood pressure is determined as a product of $K_{RD} * K_{nD} * K_{indD} * ((U911)*S)$ which means $K_{RD} * K_{nD} * K_{indD} * (\sqrt{((Tx9-Tx11)^2 + (Ty9-Ty11)^2)} * S)$, together with determining of the diastolic blood pressure as a result of $K_{RD} * K_{nD} * K_{indD}$

*((TyST*TxST)*S) and/or $K_{RD}$ * $K_{nD}$ * $K_{indD}$ *STT, respectively $K_{RD}$ * $K_{nD}$ * $K_{indD}$ *(TySTT*TxSTT).

3. The method according to claim 1 or 2, **characterized in that** the segment (2a) must be longer than minimal a and shorter than maximal allowed limit while the limits are defined in constants of the time units equal to a number of evaluated pulses of the heart beat from 5 pulses as the minimum to 250 pulses as the maximum.

4. The method according to any of the previously mentioned claims, **characterized in that** the determining the number of selected segments (2b) used for averaging is defined as a constant ($K_{prum}$), it is determined according to a type of the scanning unit in connection to its own interference of its amplifier and it is a compromise in a sense that the signal is desirably purified and at the same time it still doesn't affect the signal significantly, so the rapid changes near the local extremes are not distorted.

5. The method according to the claim 4, **characterized in that** the value of the constant ($K_{prum}$) is typically in the range from 2 to 30 of consecutive samples.

6. The method according to any of the previously mentioned claims, **characterized in that** the matching the value Y of the global maximum (M3) with the value 1.

7. The method according to any of the previously mentioned claims, **characterized in that** the determining the value 0 as a result of the arithmetic average of the beginning (M1) and the end (M2) of the selected segment (2b) which defines the shift in the offset value, the multiplicative constant gain is defined according to the claim 6 and the selected segments (2b) are one-dimensionally transformed with the help of values offset a gain.

8. The method according to the claim 1 or 2, **characterized in that** the area of each selected segment (2b) that is divided into 6 parts defined as the dividing vertical form the maximum (M3) divided in the axis Y for values 35 % and 55 % out of maximum (M3).

9. The method according the claim 1 or 2, **characterized in that** the only selected segments (2b) are left that differ of 5 % at maximum.

10. The method according to claims 1 or 2 **characterized in that** the values of the plethysmografic curve (1) for selected segments (2b) with the difference lower than 25 % that are recalculated so the beginning and the end of minimums of each selected segment (2b) have the same value and the recalculated segments (2b) are transformed using the values offset and gain.

11. The method according to claims 1 or 2, **characterized in that** the area of each selected segment (2b) is divided into 6 parts defined as the dividing vertical out of the maximum (M3) divided in the axis Y for values 35 % and 55 % out of the maximum (M3), then areas and centers of gravity for all 6 parts are determined and from the points of centers of gravity new closed areas and/or lines are created and further there are determined total areas and centers of gravity for the selected segment (2b) as well as the area and the center of gravity of the new area created from centers of the partial areas of the segment and/or a size of the line determined from partial points of centers and/or the same in combination with the center of gravity of the whole plethysmographic curve (1).

12. A unit to operate the method according to any of the previously mentioned claims formed by the unit for monitoring and measuring the life functions of a human, comprising a superordinated controlling unit (32) configured to perform the method according to claim 1 and a supporting construction in a shape of a ring (29) and/or an earring (39) supplied with the transmitting sensor (21), other transmitting sensor (22), scanning sensor (23) for the transmitting sensor (21) and another transmitting sensor (22), and scanning sensor (24) for measuring the surrounding temperature, that are connected via interface (25) to the unit (26) of the communication module, which is by means of the wireless communication (30) connected to another communication module (31) placed outside the ring (29) and/or the earring (39) and connected to the superordinated controlling unit (32), while the transmitting sensor (21), another transmitting sensor (22), scanning sensor (23), scanning sensor (24) and the unit (26) of the communication module are powered from the battery (27) built-in the ring (29) and/or earring (39), which is attachable to a finger (28) and/or to the earlobe (38) of the scanned and measured subject, where the transmitting sensor (21) is fitted with the radiation supply working in the infrared area of the spectrum and/or other transmitting sensor (22) is fitted with the radiation supply in the red range of the spectrum, for the supply of the plethysmografic curve (1) data of the measured subject scanned by the scanning sensor (23).

**13.** The unit according to the claim 12, **characterized in that** the transmitting sensor (21) that is equipped with the LED radiation supply working in the infrared range of the spectrum.

**14.** The unit according to the claim 12, **characterized in that** the transmitting sensor (22) that is equipped with the LED radiation supply working in the red range of the spectrum.

**15.** The unit according to the claims 12 and 14, **characterized in that** the communication module (26) and/or another communication module (31) is formed by the module Bluetooth working according to the standard.

**Patentansprüche**

**1.** Das Verfahren zur Bestimmung des systolischen und diastolischen Blutdrucks, *gekennzeichnet dadurch, dass* die Datei der erhaltenen Daten einzelner Kurven der plethysmographischen Kurve (1) in einzelne Segmente (2a) unterteilt wird, mit zwei lokalen Minima, gegeben durch den Anfang (M1) und das Ende (M2) und ein globales Maximum (M3), entsprechend einem Puls des zu messenden Subjekts und darstellend einen Zyklus der Herzaktivität, woraufhin der Verlauf der Segmente (2a) der plethysmographischen Kurve (1) in einzelne Abschnitte unterteilt wird, die mindestens ein Segment (2a) mit Teilung in Abschnitten in 20 Segmenten (2a) enthalten und zur Eliminierung von gesättigtem Signal von weiterer Auswertung werden sie nach Konstante $K_a$ des gesättigten Teils der Segmente (2a) der plethysmographischen Kurve (1) von der Weiterverarbeitung ausgeschlossen, wobei die empfohlenen Werte immer nach Typ der Sensoreinheit wie Konstante $K_a$ respektive ihre Grenzwerte bestimmt werden, also $K_{aMIN}$ und $K_{aMAX}$., woraufhin der Verlauf der Segmente (2a) der plethysmographischen Kurve (1) durch Mittelwert-Bewertung aufeinanderfolgender Werte der Proben der plethysmographischen Kurve (1) gefiltert wird, bei jedem Segment (2a) wird die Differenz zwischen den Amplituden jedes Segments am Anfang (M1) und am Ende (M2) der Kurve überprüft und es verbleiben die ausgewählten Segmente (2b), die sich nicht um mehr als 10 % unterscheiden, dann werden die minimalen und maximalen Hauptmaximalwerte in Bezug auf die Sättigung des Signals mit Konstante (Ka) bestimmt, die ausgewählten Segmente (2b) werden so normiert, so dass dem Wert (Y) des globalen Maximums (M3) der Wert 1 zugeordnet wird und der Wert 0 als Ergebnis des arithmetischen Durchschnitts vom Anfang (M1) und Ende (M2) und Ende (M2) für ausgewählte Segmente (2b) mit Differenz weniger als 5 % bestimmt wird und/oder es werden die Werte der plethysmographischen Kurve (1) für ausgewählte Segmente (2b) mit Differenz weniger als 25 % so umgerechnet, so dass der Anfang und das Ende der Minima jedes ausgewählten Segments (2b) den gleichen Wert haben und die neu berechneten ausgewählten Segmente (2b) erneut linear transformiert werden, indem die Fläche jedes ausgewählten Segments (2b) in 6 Teile unterteilt wird, und dies unter der Kurve des Segments, definiert durch die Aufteilung des Segments zum Zeitpunkt des Maximums (M3) und durch die Aufteilung der Segmentamplitude in zwei Teile zwischen 20 bis 60 % und 40 bis 90 % von Maximum (M3), wobei die zweite Teillinie prozentuell größer als die erste sein muss, und es werden Teilflächen S6, S7, S8, S9, S10, S11, und der Schwerpunkt Tx6, Ty6, Tx7, Ty7, Tx8, Ty8, Tx9, Ty9, Tx10, Ty10, Tx11, Ty11 jedes Teils und der Schwerpunkt Tx, Ty, des ganzen ausgewählten Segments (2b) bestimmt, zugleich werden Konstanten $K_{indS}$ und $K_{indD}$ als statistischer Mittelwert der Kontrollmessungen bei Subjekten für systolischen und diastolischen Blutdruck bestimmt, sowie Konstanten $K_{nS}$ und $K_{nD}$ als statistische Mittelwerte der Kontrollmessungen bei Subjekten, aufgeteilt nach Alter und Geschlecht, und dies zusammen mit der Umfangsbestimmung der Konstanten $K_{RS}$ und $K_{RD}$ der Messungen und es wird die Größe D als erste Ableitung der Vorderkante jedes Segments (2a) bestimmt und jetzt wird der systolische Blutdruck als Produkt von $K_{RS} * K_{nS} * K_{indS} *D*S8$ und der diastolische Blutdruck als Produkt von $K_{RD} * K_{nD} * K_{indD} * ((Ty*Tx)*S)$ bestimmt.

**2.** Das Verfahren zur Bestimmung des systolischen und diastolischen Blutdrucks nach Anspruch 1, *gekennzeichnet dadurch, dass* Teilflächen (S6, S7, S8, S9, S10, S11) und der Schwerpunkt (Tx6, Ty6, Tx7, Ty7, Tx8, Ty8, Tx9, Ty9, Tx10, Ty10, Tx11, Ty11) jedes Teils und der Schwerpunkt (Tx, Ty) des ganzen ausgewählten Segments (2b) der plethysmographischen Kurve (1) bestimmt wird und mit Hilfe von diesen werden neue Flächen (ST67810) mit dem Schwerpunkt (TxST67810, TyST67810) und/oder Linie (U911) und/oder mit Gesamtschwerpunkt (Tx, Ty), Flächen (STT67810) und (STT) mit dem Schwerpunkt (TxSTT67810, TySTT67810) und (TxSTT, TySTT) bestimmt und weiterhin wird der systolische Blutdruck als Ergebnis von $K_{RS} * K_{nS} * K_{indS} *D*ST67810$ respektive $K_{RS} * K_{nS} * Kinds *D*(TyST67810* TxST67810)$ bestimmt und gleichzeitig der diastolische Blutdruck als Ergebnis von $K_{RD} * K_{nD} * K_{indD} *((U911)*S)$ also in Aufschlüsselung $K_{RD} * K_{nD} * K_{indD} *(\sqrt{((Tx9-Tx11)^2+(Ty9-Ty11)^2)} * S)$, zusammen mit der Bestimmung des diastolischen Blutdrucks als Ergebnis von $KRD * K_{nD} * K_{indD} *((TyST*TxST)*S)$ und/oder $K_{RD} * K_{nD} * K_{indD} *STT$, respektive $K_{RD} * K_{nD} * K_{indD} *(TySTT*TxSTT)$ bestimmt.

**3.** Das Verfahren nach Anspruch 1 oder 2, *gekennzeichnet dadurch, dass* das Segment (2a) länger als der minimale und kürzer als der maximale Grenzwert sein muss, wobei die Grenzwerte in Zeiteinheitskonstanten äquivalenter Anzahl bewerteter Herzfrequenzimpulse von 5 Pulse als Minimum bis 250 Pulse als Maximum definiert werden.

4. Das Verfahren nach einem der obigen Ansprüche, *gekennzeichnet dadurch, dass* die Bestimmung von ausgewählten Segmenten (2b), verwendet bei Ermittlung der Mittelwerte, als Konstante ($K_{prum}$) definiert wird, die nach dem Typ der Sensoreinheit in Bezug auf eigenes Rauschen deren Verstärkers bestimmt wird und einen Kompromiss in dem Sinne darstellt, dass das Signal wie gewünscht entfernt wird und zugleich das Signal noch nicht unerwünscht verzerrt wird, insbesondere erfolgen dessen schnellere Änderungen in Umgebung lokaler Extreme.

5. Das Verfahren nach Anspruch 4, *gekennzeichnet dadurch, dass* der Wert der Konstante ($K_{prum}$) typischerweise im Bereich von 2 bis 30 aufeinanderfolgenden Proben liegt.

6. Das Verfahren nach einem der obigen Ansprüche, *gekennzeichnet dadurch, dass* dem Y-Wert des globalen Maximums (M3) der Wert 1 zugeordnet wird.

7. Das Verfahren nach einem der obigen Ansprüche, *gekennzeichnet dadurch, dass* durch die Bestimmung des Wertes 0 als Ergebnis des arithmetischen Mittelwertes vom Anfang (M1) und Ende (M2) des ausgewählten Segments (2b) die Verschiebung im Wert Offset definiert wird und die Mehrfachkonstante Gain nach Anspruch 6 definiert wird und die ausgewählten Segmente (2b) linear mit Hilfe von Werten Offset und Gain transformiert werden.

8. Das Verfahren nach Anspruch 1 oder 2, *gekennzeichnet dadurch, dass* die Fläche jedes ausgewählten Segments (2b) in 6 Teile aufgeteilt wird, definiert als teilende Senkrechte von Maximum (M3), geteilt in Y-Achse für Werte 35 % und 55 % vom Maximum (M3).

9. Das Verfahren nach Anspruch 1 oder 2, *gekennzeichnet dadurch, dass* nur ausgewählte Segmente (2b) verbleiben, die sich maximal um 5 % unterscheiden.

10. Das Verfahren nach Anspruch 2, *gekennzeichnet dadurch, dass* die Werte der plethysmographischen Kurve (1) für ausgewählte Segmente (2b) mit Differenz weniger als 25 %, so umgerechnet werden, so dass der Anfang und das Ende der Minima jedes ausgewählten Segments (2b) den gleichen Wert haben und die neu berechneten ausgewählten Segmente (2b) linear mit Hilfe von Werten Offset und Gain transformiert werden.

11. Das Verfahren nach Anspruch 1 oder 2, *gekennzeichnet dadurch, dass* die Fläche jedes ausgewählten Segments (2b) in 6 Teile unterteilt wird, definiert als teilende Senkrechte von Maximum (M3) geteilt in Y-Achse für Werte 35 % und 55 % vom Maximum (M3), woraufhin für alle 6 Teile deren Inhalte und Schwerpunkte bestimmt werden, und es werden aus Schwerpunkten weitere geschlossene Flächen und/oder Linien gebildet und weiterhin werden Gesamtflächen und Schwerpunkte für ausgewähltes Segment bestimmt, sowie die Fläche und der Schwerpunkt neuer Fläche, gebildet aus Schwerpunkten von Teilflächen des Segments und/oder die Größe der Linie, bestimmt von Teilschwerpunkten und/oder dasselbe in Kombination mit dem Schwerpunkt der gesamten plethysmographischen Kurve (1).

12. Die Einheit zur Durchführung vom Verfahren nach einem der obigen Ansprüche, gebildet aus Einheit zur Überwachung und Messung der Vitalfunktionen des Menschen, umfassend eine übergeordnete Steuereinheit (32), eingestellt zur Durchführung des Verfahrens nach Anspruch 1, und eine Tragkonstruktion in Form vom Ring (29) und/oder Ohrring (39), versehen mit einem Sendesensor (21), einem weiteren Sendesensor (22), einem Erfassungssensor (23) für Sendesensor (21) und weiteren Sendesensor (22), und einem Erfassungssensor (24) zur Messung der Umgebungstemperatur, die über eine Schnittstelle (25) an der Einheit (26) des Kommunikationsmoduls angeschlossen sind, die durch eine drahtlose Kommunikation (30) an einem weiteren Kommunikationsmodul (31) angeschlossen ist, platziert außerhalb des Rings (29) und/oder des Ohrrings (39) und verbunden mit der übergeordneten Steuereinheit (32), wobei der Sendesensor (21), der weitere Sendesensor (22), der Erfassungssensor (23), der Erfassungssensor (24) und die Einheit (26) des Kommunikationsmoduls von Batterie (27) gespeist werden, integriert im Ring (29) und/oder Ohrring (39), der auf dem Finger (28) und/oder Ohrläppchen (38) des gemessenen Subjektes aufsetzbar ist, wobei der Sendesensor (21) mit einer Strahlungsquelle im Infrarotspektrum versehen ist und/oder der weitere Sendesensor (22) mit einer Strahlungsquelle im Bereich des Infrarotspektrums für eine Datenquelle der plethysmographischen Kurve (1) des gemessenen Subjektes versehen ist, erfasst mit dem Erfassungssensor (23).

13. Die Einheit nach Anspruch 12, *gekennzeichnet dadurch, dass* der Sendesensor (21) mit einer Strahlungsquelle in LED-Form im Bereich des Infrarotspektrums versehen ist.

**14.** Die Einheit nach Anspruch 12, *gekennzeichnet dadurch, dass* der weitere Sendesensor (22) mit einer Strahlungsquelle in LED-Form im Bereich des Infrarotspektrums versehen ist.

**15.** Die Einheit nach Anspruch 12 bis 14, *gekennzeichnet dadurch, dass* das Kommunikationsmodul (26) und/oder das weitere Kommunikationsmodul (31) aus Standard-Bluetooth-Modul gebildet wird.

**Revendications**

**1.** Procédé de détermination de la pression artérielle systolique et diastolique, *caractérisé en ce que* l'ensemble des données obtenues des différentes courbes de la courbe (1) pléthysmographique est divisé en segments (2a) individuels avec deux minimums locaux donnés par le début (M1) et la fin (M2) et un maximum global (M3) correspondant à un pouls du sujet suivi et représentant un cycle d'activité cardiaque, l'évolution des segments (2a) de la courbe (1) pléthysmographique étant ensuite divisée en sections comprenant au moins un segment (2a) dont les sections sont divisées en 20 segments (2a) et, pour exclure le signal de saturation de toute évaluation ultérieure, selon la constante $K_a$, les partie saturées des segments (2a) de la courbe (1) pléthysmographique sont enlevées du traitement ultérieur, les valeurs recommandées étant toujours déterminées en fonction du type d'unité de détection, comme la constante $K_a$ ou sa limite, c.-à-d. $K_{aMIN}$ a $K_{aMAX}$·, l'évolution des segments (2a) de la courbe (1) pléthysmographique étant filtrée ensuite en faisant la moyenne des valeurs consécutives des échantillons de la courbe (1) pléthysmographique pour chaque segment (2a), les différences entre les amplitudes de chaque segment au début (M1) et à la fin (M2) de la courbe sont vérifiées, ne laissant que les segments (2b) sélectionnés qui diffèrent au maximum de 10%, puis les valeurs minimale et maximale du maximum principal par rapport à la saturation du signal avec la constante (Ka) sont déterminées, les segments (2b) sélectionnés sont normalisés de sorte qu'à la valeur (Y) du maximum global (M3) la valeur 1 est attribuée et la valeur 0 est déterminée comme la moyenne arithmétique du début (M1) et de la fin (M2) de la fin (M2) pour les segments (2b) sélectionnés avec une différence inférieur à 5% et/ou les valeurs de la courbe (1) pléthysmographique pour les segments (2b) sélectionnés avec une différence inférieure à 25% sont recalculées de sorte que le début et la fin des minima de chaque segment (2b) sélectionné aient la même valeur et les segments (2b) sélectionnés recalculés se transforment à nouveau de manière linéaire, la surface de chaque segment (2b) sélectionné sera ensuite divisée en 6 parties sous la courbe du segment défini par la division du segment dans le temps au point maximum (M3) et par la division de l'amplitude du segment en deux parties entre 20 et 60% et 40 à 90% du maximum (M3), la deuxième droite de division doit être, en pourcentage, supérieure à la première, et les surfaces S6, S7, S8, S9, S10, S11 partielles seront déterminées ainsi que le centre de gravité Tx6, Ty6, Tx7, Ty7, Tx8, Ty8, Tx9, Ty9, Tx10, Ty10, Tx11, Ty11 de chaque partie et du centre de gravité Tx, Ty de l'ensemble du segment (2b) sélectionné, les constantes $K_{indS}$ a $K_{indD}$ sont également déterminées comme valeurs de moyenne statistique des échantillons de mesures de contrôle des sujets pour la pression systolique et diastolique et les constantes $K_{nS}$ a $K_{nD}$ comme valeurs de moyenne statistique des mesures de contrôle du sujet, divisées selon classe d'âge et le sexe, et ce, ensemble avec la détermination des constantes de la plage de mesure $K_{RS}$ a $K_{RD}$ de la plage de mesures et la valeur D sera déterminée comme la première dérivée du bord d'attaque de chaque segment (2a) et la pression systolique est maintenant définie comme le produit de $K_{RS}$ * $K_{nS}$ * $K_{indS}$ *D * S8 et la pression diastolique comme le produit $K_{RD}$ * $K_{nD}$ * $K_{indD}$ * ((Ty*Tx)*S).

**2.** Procédé de détermination de la pression artérielle systolique et diastolique selon la revendication 1, *caractérisé en ce que* les surfaces (S6, S7, S8, S9, S10, S11) partielles et le centre de gravité (Tx6, Ty6, Tx7, Ty7, Tx8, Ty8, Tx9, Ty9, Tx10, Ty10, Tx11, Ty11) de chaque partie et le centre de gravité (Tx, Ty) de l'ensemble du segment (2b) sélectionné de la courbe (1) pléthysmographique seront déterminés et à l'aide de ceux-ci, de nouvelles surfaces (ST67810) avec son centre de gravité (TxST67810, TyST67810) et/ou la droite (U911) et/ou avec le centre de gravité général (Tx,Ty), les surfaces (STT67810) et (STT) et leurs centres de gravité (TxSTT67810, TySTT67810) et (TxSTT, TySTT), ensuite la pression artérielle systolique sera déterminée comme le résultat du $K_{RS}$ * $K_{nS}$ * $K_{indS}$ *D*ST67810 ou $K_{RS}$ * $K_{nS}$ * $K_{indS}$ *D*(TyST67810* TxST67810) et la pression artérielle diastolique sera déterminée comme le résultat du $K_{RS}$ * $K_{nS}$ * $K_{indS}$ *D*ST67810 ou $K_{RS}$ * $K_{nS}$ * $K_{indS}$ *D*(TyST67810* TxST67810) et la pression artérielle diastolique sera déterminée comme le résultat $K_{RD}$ * $K_{nD}$ * $K_{indD}$ *((U911)*S) donc en détails $K_{RD}$ * $K_{nD}$ * $K_{indD}$ *($\sqrt{((Tx9-Tx11)^2 + (Ty9-Ty11)^2)}$ * S), avec la détermination la pression artérielle diastolique comme le résultat de $K_{RD}$ * $K_{nD}$ * $K_{indD}$ * ((TyST*TxST)*S) et/ou $K_{RD}$ * $K_{nD}$ * $K_{indD}$ *STT, ou $K_{RD}$ * $K_{nD}$ * $K_{indD}$ *(TySTT*TxSTT).

**3.** Procédé selon la revendication 1 ou 2, *caractérisé en ce que* le segment (2a) doit être plus long que la limite minimale admise et plus court que la limite maximale admise, les limites étant définies en constantes d'unités de temps équivalentes au nombre d'impulsions de fréquence cardiaque évaluées, de 5 impulsions au minimum à 250 impulsions au maximum.

**4.** Procédé selon l'une quelconque des revendications précédentes, ***caractérisé en ce que*** la détermination des segments (2b) sélectionnés utilisés pour calculer la moyenne est défini comme une constante ($K_{prum}$) elle-même déterminée en fonction du type de l'unité de détection en lien avec les parasites propres à son amplificateur et représente un compromis en ce sens que le signal souhaité est lissé et qu'aucune distorsion significative indésirable du signal, en particulier ses changements plus rapides autour des extrêmes locaux, n'existe pas encore.

**5.** Procédé selon la revendication 4, ***caractérisé en ce que*** la valeur de la constante ($K_{prum}$) se situe typiquement entre 2 et 30 échantillons consécutifs.

**6.** Procédé selon l'une quelconque des revendications précédentes ***caractérisé en ce qu'***à la valeur du maximum global (M3), la valeur 1 est attribuée.

**7.** Procédé selon l'une quelconque des revendications précédentes ***caractérisé en ce que*** le décalage en offset est défini en déterminant la valeur 0 comme la moyenne arithmétique du début (M1) et de la fin (M2) du segment (2b) sélectionné et la constante multiple gain est définie selon la revendication 6 et les segments (2b) sélectionnés sont transformés de manière linéaire à l'aide des valeurs offset et gain.

**8.** Procédé selon la revendication 1 ou 2, ***caractérisé en ce que*** la surface de chaque segment (2b) sélectionné est divisée en 6 parties définies comme la perpendiculaire de partage du maximum (M3) divisée sur l'axe Y pour les valeurs 35% et 55% du maximum (M3).

**9.** Procédé selon la revendication 1 ou 2, ***caractérisé en ce que*** seuls les segments (2b) sélectionnés qui diffèrent au maximum de 5% seront conservés.

**10.** Procédé selon la revendication 2, ***caractérisé en ce que*** les valeurs de la courbe (1) pléthysmographique pour les segments (2b) sélectionnés avec une différence de moins de 25% seront recalculés de sorte que le début et la fin des minima de chaque segment (2b) sélectionné aient la même valeur et les segments (2b) sélectionnés recalculés se transforment à nouveau de manière linéaire à l'aide des valeurs offset et gain.

**11.** Procédé selon la revendication 1 ou 2, ***caractérisé en ce que*** la surface de chaque segment (2b) sélectionné sera ensuite divisée en 6 parties définies comme la perpendiculaire de partage du point maximum (M3) divisée sur l'axe Y pour les valeurs 35% et 55% du maximum (M3), ensuite les contenues pour les 6 parties seront déterminés ainsi que leurs centres de gravité et, à partir des points des centres de gravité, d'autres surfaces fermées ou une droite seront crées et ensuite les surfaces totales et le centre de gravité pour le segment sélectionné seront déterminées ainsi que la surface et le centre de gravité de la nouvelle surface crée à partir des centres de gravité des surfaces partielles du segment et/ou la taille de la droite déterminé à partir des points partiels des centres de gravité et/ou la même chose en combinaison avec le centre de gravité de la totalité de la courbe (1) pléthysmographique.

**12.** Unité pour l'exécution du procédé selon l'une quelconque des revendications précédentes constituée de l'unité de suivi et de mesure des fonctions vitales de l'homme, comprenant une unité (32) de commande supérieure ajustée pour réaliser le procédé selon la revendication 1 et une structure porteuse en forme de bague (29) et/ou de boucle d'oreille (39) munie d'un capteur (21) émetteur, d'un autre capteur (22) émetteur, d'un capteur (23) de détection pour le capteur (21) émetteur et l'un autre capteur (22) émetteur, et d'un capteur (24) pour mesurer la température ambiante, ceux-ci sont connectés via l'interface (25) à l'unité du module (26) de communication qui est connectée via communication (30) sans fil à un autre module (31) de communication situé à l'extérieur de la bague (29) et/ou de la boucle d'oreille (39) et connecté à une unité de commande supérieure (32), le capteur (21) émetteur, l'autre capteur (22) émetteur, le capteur (23) de détection, le capteur (24) de détection et l'unité (26) du module de communication sont alimentés par une batterie (27) intégrée au bague (29) et/ou à la boucle d'oreille (39) engageables sur un doigt (28) et/ou un lobe de l'oreille (38) du sujet surveillé et mesuré, le capteur (21) émetteur étant muni d'une source de rayonnement dans la zone infrarouge du spectre et/ou l'autre capteur (22) émetteur étant muni d'une source de rayonnement dans la zone rouge du spectre, pour la source de données de la courbe (1) pléthysmographique du sujet mesuré enregistrée par le capteur (23) de détection.

**13.** Unité selon la revendication 12, ***caractérisé en ce que*** le capteur (21) émetteur est muni d'une source de rayonnement sous forme de LED fonctionnant dans la zone infrarouge du spectre.

**14.** Unité selon la revendication 12, ***caractérisé en ce que*** l'autre capteur (22) émetteur est muni d'une source de rayonnement sous forme de LED fonctionnant dans la zone infrarouge du spectre.

**15.** Unité selon la revendication 12 à 14, ***caractérisé en ce que*** le module (26) de communication et/ou l'autre module (31) de communication est constitué d'un module Bluetooth fonctionnant selon la norme.

**Fig. 1**

**Fig. 2**

**Fig. 3**

**Fig. 4**

Fig. 5

Fig. 6

**Fig. 7**

**Fig. 8**

**Fig. 9**

**Fig. 10**

wrist band/ring/earring

Visualization element (button)
Alternatively: keyboard

Modules - processing and measurement:
- Temperature
- O2 saturation
- Systolic/diastolic pressure
- Pulse - Hearth Rate
- Breath
- GNSS (GPS/GALILEO/GLONASS ...)
- Requested measurements (ECG ...)
- Comm. nanoworld-macroworld
- Comm with applied apparatus
- Other nets (802.XX - as needed)
- 3D accelerometer

Visualization element (LED)
Alternatively Display

Alternatively Modul of special comm.

Alternatively Standard mobile communication (GSM/GPRS/EDGE UMTS, LTE)

Bluetooth module

**Communication scheme**

**Home center**

Nanobots

**Applied apparatus**

GPS

**Emergency center**

SmartPhone
GSM/GPRS/EDGE UMTS, LTE
GNSS - GPS/GLONASS/GALILEO

Central care

EP 3 010 400 B1

Description of Fig. 8 and Fig.9:

21     transmitting sensor s1

22     transmitting sensor s2

23     scanning sensor for s1 and s2

24     scanning sensor for the surrounding temperature 28

25     interface for a connection of the communication module 26 to sensors 21, 22, 23, 24

26     communication module

27     battery

28     interior of the ring 29 (finger)

29     ring – supporting construction

30     wireless communication of modules 26 and 31

31     communication module

32     controlling, assessment and computing unit

38     earlobe

39     ear – supporting construction

**EP 3 010 400 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009326393 A